# EUROPEAN PATENT APPLICATION

(11) **EP 4 394 378 A1**
(43) Date of publication of application: **03.07.2024**
(21) Application number: 22879900.3
(22) Date of filing: 11.07.2022
(51) Int. Cl.: G01N 33/48

(54) **SENSOR AND METHOD FOR OBTAINING ANALYTE CONCENTRATION WHILE TAKING INTO CONSIDERATION TEMPERATURE COMPENSATION**

(30) Priority: 12.10.2021 CN 202111187930
(71) Applicant: Shenzhen Sisensing Technology Co., Ltd., Shenzhen, Guangdong 518000 (CN)
(72) Inventor: CHEN, Zhi, Shenzhen, Guangdong 518000 (CN); LIU, Shishan, Shenzhen, Guangdong 518000 (CN); PENG, Weibin, Shenzhen, Guangdong 518000 (CN); FANG, Junfei, Shenzhen, Guangdong 518000 (CN); GONG, Mingli, Shenzhen, Guangdong 518000 (CN); HAN, Mingsong, Shenzhen, Guangdong 518000 (CN)
(74) Representative: Isern Patentes y Marcas S.L.
(86) International application number: PCT/CN2022/104810
(87) International publication number: WO 2023/060953

(57) **Abstract**

The present disclosure provides a method for obtaining an analyte concentration with temperature compensation considered, comprising: obtaining sensitivity information of the implanted part at a predetermined analyte concentration prior to an analyte sensor being worn, the sensitivity information is a relationship between the temperature and the sensitivity of the implanted part; placing the implanted part subcutaneously and the application part on a body surface, obtaining a body surface temperature via the temperature sensor, and obtaining a subcutaneous temperature based on the body surface temperature; selecting a reference temperature; obtaining calibration information based on the sensitivity information, the reference temperature, and the subcutaneous temperature, and calibrating a response signal obtained by the implanted part based on the calibration information; and obtaining the analyte concentration based on the reference temperature and the calibrated response signal. Thereby, the accuracy of the obtained analyte concentration can be improved.

## Description

### BACKGROUND OF INVENTION

### Field of Invention

The present disclosure substantially relates to the field of medical devices, and more particularly to a sensor and a method for obtaining an analyte concentration with temperature compensation considered.

### Description of Related Art

Diabetes mellitus is disease caused by a series of metabolic disorders such as sugar, protein, fat, water and electrolytes, which may incur complications if not well controlled, for example, ketoacidosis, lactic acidosis, chronic renal failure and retinopathy. For diabetic patients, if the glucose concentration can be monitored continuously in real time, the occurrence of complications such as glycosemia and hyperglycemia can be predicted preferentially.

Studies have shown once the glucose concentration in the blood begins to decrease, the glucose concentration in the interstitial fluid decreases earlier than the glucose concentration in the blood does. Therefore, the decrease in the glucose concentration in the interstitial fluid can be predictive of an impending low glucose concentration. A glucose sensor for sensing glucose concentration generally includes an implanted part that is capable of being placed subcutaneously to sense changes in glucose concentration in subcutaneous tissue fluid to enable prediction of glucose concentration in the blood.

However, the temperature in the location of the implanted part is subject to a combination of changes in environment temperature and changes in body temperature. The implanted part generally includes an enzyme that catalyzes the reaction of glucose. Since the activity of the enzyme is affected by temperature, deviation of the response signal output from the implanted part may result in inaccurate calculation of the glucose concentration based on the response signal. It is therefore necessary to calibrate the output response signal with temperature compensation considered.

### SUMMARY OF INVENTION

The present disclosure has been made in view of the above-mentioned state of the art, and an object thereof is to provide a sensor and a method for obtaining an analyte concentration with temperature compensation considered, so as to improve the accuracy of the obtained analyte concentration.

For the above purpose, the present disclosure provides a method for obtaining analyte concentration with temperature compensation considered, the analyte concentration is obtained by an analyte sensor, the analyte sensor includes an implanted part capable of being placed subcutaneously and an application part capable of being placed on a body surface and having a temperature sensor. The method includes: obtaining sensitivity information of the implanted part at a predetermined analyte concentration prior to the analyte sensor being worn, the sensitivity information is a relationship between temperature and sensitivity of the implanted part; placing the implanted part subcutaneously and the application part on a body surface to obtain a body surface temperature via the temperature sensor, and obtaining a subcutaneous temperature based on the body surface temperature; selecting a reference temperature; obtaining calibration information based on the sensitivity information, the reference temperature, and the subcutaneous temperature, and calibrating a response signals obtained by the implanted part based on the calibration information; and obtaining the analyte concentration based on the reference temperature and the calibrated response signals.

In the method of the present disclosure, prior to wearing the analyte sensor, the sensitivity information of the implanted part is obtained at a predetermined analyte concentration, i.e., relationship between the sensitivity of the implanted part and the temperature is obtained. The implanted part is placed subcutaneously and the application part having a temperature sensor is placed on the body surface. The body surface temperature is obtained from the temperature sensor, and a subcutaneous temperature is obtained based on the body surface temperature. The reference temperature is selected, and based on the sensitivity information, the reference temperature, and the subcutaneous temperature, the calibration information is obtained. The response signal obtained from the implanted part is calibrated based on the calibration information, whereby a calibrated response signal can be obtained. Based on the reference temperature, as well as the calibrated response signals, the analyte concentration is calibrated, whereby the accuracy of the obtained analyte concentration can be improved.

Additionally, in the method of the present disclosure, optionally, the method comprises placing the implanted part in a reagent containing the analyte, changing a temperature of the reagent, and measuring a variation of a sensitivity of the implanted part over the temperature of the reagent to obtain sensitivity information of the implanted part. In this case, prior to the analyte sensor being worn, it is facilitated for the sensitivity information of the implanted part to be obtained in advance by measuring the variation of sensitivity of the implanted part over temperature using a reagent containing the analyte.

Additionally, in the method of the present disclosure, optionally, the method comprises keeping the concentration of the analyte in the reagent to be constant as changing the temperature of the reagent. In this case, through controlling the concentration of the analyte in the reagent to be constant and changing the temperature of the reagent, the relationship between the sensitivity of the implanted part and the temperature can be obtained more accurately.

Additionally, in the method of the present disclosure, optionally, the method comprises obtaining the calibration information based on the sensitivity information and a difference or ratio of the subcutaneous temperature to the reference temperature. In this case, with the consideration of relationship between the sensitivity of the implanted part and the temperature, and the relationship between the subcutaneous temperature of the location of the implanted part and the reference temperature, the temperature compensation can be facilitated.

Additionally, in the method of the present disclosure, optionally, once the temperature sensor senses a temperature on the body surface and outputs the body surface temperature, the implanted part inserted subcutaneously senses a subcutaneous analyte concentration simultaneously and outputs response signals. In this case, the subcutaneous temperature, i.e. the temperature in the location of the implanted part, and the response signal output by the implanted part sensing the analyte concentration can be simultaneously obtained. Thus, the temperature compensation can be made for the response signal of the implanted part in real time to improve the accuracy of the analyte concentration calibration.

Additionally, in the method of the present disclosure, optionally, there is no time delay between the output of the response signal by the implanted part and the output of the body surface temperature by the temperature sensor. The accuracy of analyte concentration calibration can thereby be improved.

Additionally, in the method of the present disclosure, optionally, the analyte is one or more of acetylcholine, amylase, bilirubin, cholesterol, chorionic gonadotropin, creatine kinase, creatine, creatinine, DNA, fructosamine, glucose, glutamine, growth hormone, hormone, ketone body, lactate, peroxide, prostate specific antigen, prothrombin, RNA, thyroid stimulating hormone, and troponin. Thus, the concentration of analytes such as acetylcholine, amylase, and bilirubin can be obtained.

In addition, in the method of the present disclosure, optionally, the method comprises obtaining a calibrated analyte concentration based on the relationship between the response signal of the implanted part and the change in analyte concentration at the reference temperature and the calibrated response signal.

The present disclosure also provides an analyte sensor for obtaining an analyte concentration with temperature compensation considered, the analyte sensor includes an implanted part capable of being placed subcutaneously, an application part capable of being placed on a body surface and has a temperature sensor, and a processing module storing a first mapping relationship between the subcutaneous temperature and the temperature of the body surface obtained by the temperature sensor, sensitivity information of the implanted part, and a second mapping relationship between response signals output by the implanted part and change in the analyte concentration at a predetermined temperature, wherein the sensitivity information is a relationship between a sensitivity of the implanted part and a temperature change at a predetermined analyte concentration; when the implanted part is placed subcutaneously and the application part is placed on the body surface, the temperature sensor senses the temperature of the body surface and outputs the temperature of the body surface; and the processing module is configured for obtaining a subcutaneous temperature based on the body surface temperature and the first mapping relationship, selecting a reference temperature, obtaining calibration information based on the sensitivity information, the subcutaneous temperature, and the reference temperature, calibrating response signals obtained from the implanted part based on the calibration information, and obtaining the analyte concentration based on the reference temperature, the calibrated response signals, and the second mapping relationship.

According to the analyte sensor of the present disclosure, the implanted part is placed subcutaneously and the application part having a temperature sensor is placed on the body surface. The body surface temperature is obtained from the temperature sensor, and a subcutaneous temperature is obtained based on the body surface temperature. The processing module is configured for obtaining a subcutaneous temperature based on the body surface temperature and the first mapping relationship, selecting a reference temperature, obtaining calibration information based on the sensitivity information, the subcutaneous temperature, and the reference temperature, calibrating a response signal obtained from the implanted part based on the calibration information, and obtaining the analyte concentration based on the reference temperature, the calibrated response signal, and the second mapping relationship. Therefore, the analyte concentration can be obtained with temperature compensation considered, improving the accuracy of analyte concentration sensed by the analyte sensor.

In addition, in the analyte sensor of the present disclosure, the implanted part includes a working electrode capable of reacting with an analyte, and a counter electrode forming a loop with the working electrode. The implanted part is thereby capable of sensing the analyte concentration.

According to the present disclosure, it is able to provide a sensor and a method for obtaining an analyte concentration with temperature compensation considered. Therefore, it is able to improve the accuracy of the obtained analyte concentration.

### BRIEF DESCRIPTION OF THE DRAWINGS

Fig. 1 is a schematic diagram showing a wearing state of an analyte sensor for obtaining an analyte concentration with temperature compensation considered according to an embodiment of the present disclosure.
Fig. 2 is a schematic structural diagram showing an implanted part of an analyte sensor according to an embodiment of the present disclosure.
Fig. 3 is a schematic structural diagram showing a working electrode of an implanted part according to an embodiment of the present disclosure.
Fig. 4 is a diagram showing a first mapping relationship between a subcutaneous temperature and a body surface temperature obtained by a temperature sensor according to an embodiment of the present disclosure.
Fig. 5A is a diagram showing results of a linear regression simulation of response current versus temperature for an implanted part according to an embodiment of the present disclosure;
Fig. 5B is a table corresponding to the linear regression simulation result diagram in Fig. 5A.
Fig. 6 is a diagram showing a second mapping relationship between response current versus analyte concentration according to an embodiment of the present disclosure.
Fig. 7 is a flowchart showing a method for obtaining an analyte concentration with temperature compensation considered according to an embodiment of the present disclosure.

### DETAILED DESCRIPTION

The present disclosure is described in further detail below with reference to the accompanying drawings and detailed description. In the drawings, the same components or components having the same functions are denoted by the same symbols, and repeated description thereof is omitted.

The present disclosure relates to a method for obtaining an analyte concentration with temperature compensation considered, which can calibrate the obtained analyte concentration with the temperature compensation considered. By the method according to this embodiment, it is able to facilitate the improvement of the accuracy of the obtained analyte concentration.

In the method for obtaining an analyte concentration with temperature compensation considered to which the present disclosure relates, the analyte concentration may be obtained by an analyte sensor. For understanding convenience, the present disclosure first introduces an analyte sensor for obtaining an analyte concentration with temperature compensation considered.

In some examples, the analyte sensor may also sometimes be referred to an implantable analyte sensor, an analyte monitor, or an analyte monitoring instrument. It should be noted that each of the names is intended to indicate an analyte sensor according to the present embodiment capable of improving the accuracy of the obtained analyte concentration with temperature compensation considered, and should not be understood to be limiting.

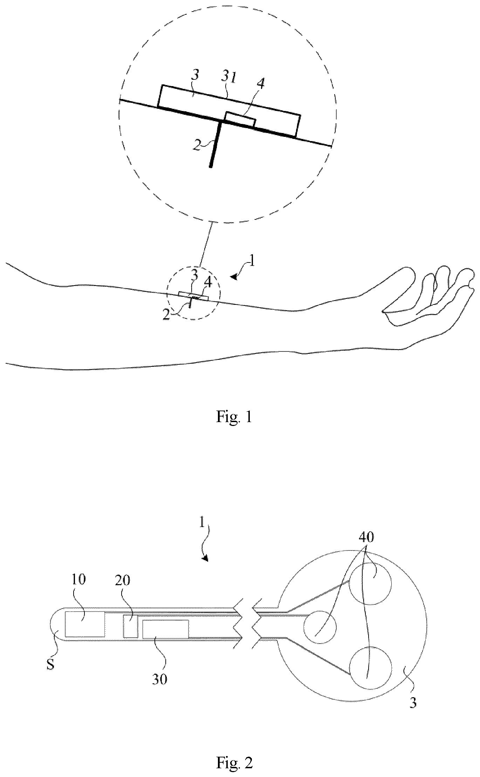

Fig. 1 is a schematic diagram showing a wearing state of an analyte sensor 1 for obtaining an analyte concentration with temperature compensation considered according to an embodiment of the present disclosure.

In some examples, the analyte sensor 1 may include an implanted part 2 that may be placed subcutaneously, an application part 3 that may be placed on a body surface, and a processing module (see Fig. 1, the processing module is not shown). In some examples, when placed subcutaneously, the implanted part 2 may sense a concentration of the analyte subcutaneously and output response signals. In some examples, the application part 3 may have a temperature sensor 4 (see Fig. 1). In some examples, when the application part 3 is placed on the body surface, the temperature sensor 4 may detect and output a temperature of the body surface. In some examples, the processing module may receive the response signals output by the implanted part 2 and the body surface temperature output by the temperature sensor 4 and calculate and output a calibrated analyte concentration.

Fig. 2 is a schematic structural diagram showing an implanted part 2 of an analyte sensor 1 according to an embodiment of the present disclosure.

In some examples, as described above, the analyte sensor 1 may include an implanted part 2 (see Fig. 1). In some examples, the implanted part 2 of the analyte sensor 1 may be placed subcutaneously and contacted with subcutaneous tissue fluid (see Fig. 1). The implanted part 2 can sense the concentration of the analyte in the interstitial fluid and output response signals.

In some examples, the implanted part 2 may be flexible. The implanted part 2 may be provided inside a puncture needle (not shown), the implanted part 2 is detachable from the puncture needle. When the analyte sensor 1 is to be worn, the puncture needle with the implanted part 2 wrapped inside may be penetrated into the tissue, then, the puncture needle is pulled out and separated from the implanted part 2, whereby the implanted part 2 is placed subcutaneously.

In some examples, the implanted part 2 may be configured in an arm (see Fig. 1), abdomen, waist, or a leg, etc.

In some examples, the implanted part 2 may be placed at 3 mm to 20 mm subcutaneously. In some examples, the depth to which the implanted part 2 is placed subcutaneously is determined according to the penetration position. When the fat layer is relatively thick, such as on the abdomen of the human body, it may be placed to a depth of about 10 mm to about 15 mm. When the fat layer is thin, such as at the arm, the depth of placement may be about 5 mm to 10 mm.

In some examples, the implanted part 2 may include a substrate S (see Fig. 2).

In some examples, the substrate S may be flexible. The substrate S may be substantially made of at least one of polyethylene (PE), polypropylene (PP), polyimide (PI), polystyrene (PS), polyethylene terephthalate (PET), and polyethylene naphtholate (PEN). In addition, in other examples, the substrate S may also be made substantially of a metal foil, ultra-thin glass, a single-layer inorganic film, a multi-layer organic film, a multi-layer inorganic film, or the like. In some examples, the substrate S may also be inflexible.

In some examples, the implanted part 2 may include a working electrode 10 and a counter electrode 30 (see Fig. 2). In some examples, the working electrode 10 may form a loop with the counter electrode 30. Thereby the implanted part 2 is able to sense the analyte concentration.

In some examples, the implanted part 2 may also include a reference and comparison electrode 20. In some examples, the implanted part 2 may also include a contact 40 connected to the working electrode 10 via a lead (see Fig. 2). Thus, the implanted part 2 can transmit response signals outward via the contact 40.

In some examples, the working electrode 10, the reference and comparison electrode 20, and the counter electrode 30 may be disposed on the substrate S (see Fig. 2).

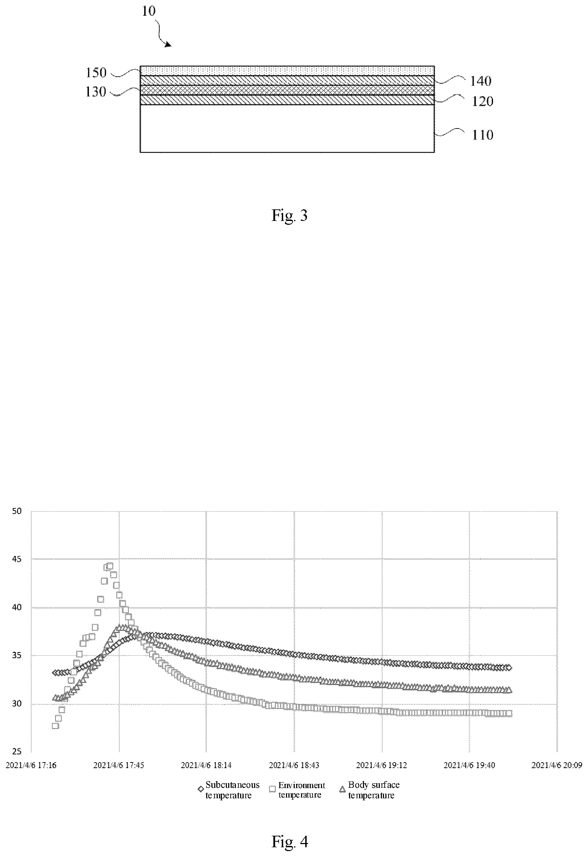

Fig. 3 is a schematic structural diagram showing a working electrode 10 of an implanted part 2 according to an embodiment of the present disclosure.

In some examples, as described above, the implanted part 2 may include a working electrode 10 (see Fig. 2). In some examples, the working electrode 10 may have a base layer 110, a nanoparticle layer 120, an analyte enzyme sensing layer 130, a semi-permeable membrane 140, and a biocompatible membrane 150. The base layer 110, the nanoparticle layer 120, the analyte enzyme sensing layer 130, the semi-permeable membrane 140, and the biocompatible membrane 150 may be sequentially laminated (see Fig. 3).

In some examples, the base layer 110 may be electrically conductive. In some examples, the base layer 110 may be made of at least one selected from gold, glassy carbon, graphite, silver, silver chloride, palladium, titanium, and iridium. In this case, the base layer 110 has good electrical conductivity and the electrochemical reaction of the base layer 110 can be suppressed, therefore, the stability of the base layer 110 can be improved.

In some examples, the base layer 110 may be disposed on the substrate S by a deposition or plating method. In some examples, methods of deposition may include physical vapor deposition, chemical vapor deposition, and the like. Methods of plating may include electroplating, chemical plating, vacuum plating, and the like. In addition, in some examples, the base layer 110 may also be provided on the substrate S through screen printing extrusion, or electrodeposition.

In some examples, the base layer 110 may be provided with an analyte enzyme sensing layer 130.

In some examples, multiple analyte concentrations may be obtained with the analyte enzyme sensing layer 130 changed on the implanted part 2. For example, in some examples, the analyte can be one or more of acetylcholine, amylase, bilirubin, cholesterol, chorionic gonadotropin, creatine kinase, creatine, creatinine, DNA, fructosamine, glucose, glutamine, growth hormone, hormone, ketone body, lactate, peroxide, prostate specific antigen, prothrombin, RNA, thyroid stimulating hormone, and troponin. In other examples, the concentration of drugs in body fluids, such as antibiotics (gentamicin, vancomycin, etc.), digoxigenin, digoxin, theophylline, warfarin, etc. may also be monitored with the analyte enzyme sensing layer 130 changed on the implanted part 2.

In some examples, the base layer 110 may be provided with a nanoparticle layer 120. That is, a nanoparticle layer 120 may be disposed between the base layer 110 and the analyte enzyme sensing layer 130. In this case, the nanoparticles can further catalyze the analyte reaction, reducing the operating voltage required by the analyte reaction and increasing the reaction rate.

Specifically, taking GOX (FAD)being glucose oxidase as an example, in the analyte sensing layer 130, when GOX (FAD) encounters glucose in the tissue, the following reaction occurs:

Glucose + GOx (FAD) - gluconolactone + GOx (FADH₂). Equation (I)

GOx (FADH₂) + O₂ → GOx (FAD) + H₂O₂. Equation (II)

In the above reaction, H₂O₂ is generated in the reaction equation (II), and the accumulation of H₂O₂ decreases the enzyme activity in the analyte enzyme sensing layer 130.

The nanoparticle layer 120 can act as a catalyst to enable H₂O₂ to be decomposed as followed:

H₂O₂→O₂+2e⁻. Equation (III)

The reaction of the implanted part 2 with glucose can be continued by the above reaction equation (I) to equation (III). In addition, by catalyzing the decomposition of hydrogen peroxide by the nanoparticle layer 120, the voltage required to be applied during the reaction is able to be reduced, facilitating the improvement of the sensitivity of the implanted part 2 and to prolong the use time of the analyte sensor 1, and to obtain a low operating voltage as well. In other words, by means of the nanoparticle layer 120, highly sensitivity sensing signals of tissue glucose can be obtained continuously, the use time of the analyte sensor 1 can be extended, and at the same time, a low operating voltage facilitates the improvement of the anti-interference performance.

In some examples, the nanoparticle layer 120 may be of porous shape. In this case, the analyte enzyme in the analyte enzyme sensing layer 130 may permeate the nanoparticle layer 120. Thus, the nanoparticle layer 120 can sufficiently contact and catalyze the reaction of the analyte so that the reaction of the analyte can be more effectively facilitated.

In some examples, the analyte enzyme may also be disposed in a three-dimensional network of conductive polymer nanofibers, i.e., the three-dimensional network of nanofibers is disposed between the nanoparticle layer 120 and the analyte enzyme sensing layer 130. Thus, the adhesion of the analyte enzyme to the nanoparticle layer 120 is increased, and the fixed amount of the analyte enzyme is increased.

In some examples, the analyte enzyme may also be disposed on carbon nanotubes. Wherein, the carbon nanotubes are disposed on the nanoparticle layer 120. Thus, the adhesiveness and the fixed amount of the analyte enzyme on the nanoparticle layer 120 are increased.

In some examples, the semi-permeable membrane 140 may be disposed on the analyte enzyme sensing layer 130. In some examples, the semi-permeable membrane 140 may further include a diffusion control layer and an anti-interference layer laminated on the diffusion control layer.

In some examples, the diffusion control layer may be disposed outside of the anti-interference layer. In the semi-permeable membrane 140, the diffusion control layer may control the diffusion of analyte molecules and the anti-interference layer may prevent the diffusion of non-analyte species. Thus, tissue fluid or blood components passing through the semi-permeable membrane 140 may be reduced first, then interfering substances are blocked outside the semi-permeable membrane 140 by the anti-interference layer. Common interfering substances may include uric acid, ascorbic acid, acetaminophen, etc. which are ubiquitous in the body. In other examples, the anti-interference layer may also be disposed outside of the diffusion control layer. Thus, it is also able to reduce inaccuracies in sensing results due to interference of impurities with the working electrode 10, and to extend the service life of the implanted part 2.

In some examples, the semi-permeable membrane 140 may control the passing rate of analyte molecules, i.e., the semi-permeable membrane 140 may limit the number of analyte molecules in the interstitial fluid or blood that reach the analyte enzyme sensing layer 130. Specifically, the diffusion control layer of the semi-permeable membrane 140 can effectively reduce the amount of the analyte diffused to the analyte enzyme sensing layer 130 in a certain proportion.

In some examples, the biocompatible membrane 150 may be disposed on the semi-permeable membrane 140. In some examples, the biocompatible membrane 150 may be made of a plant material. The plant material may be sodium alginate, gum tragacanth, pectin, arabic gum, xanthan gum, guar gum, agar, a starch derivative, or cellulose derivative, or like a derivative of a natural material. In other examples, the biocompatible membrane 150 may also be made of a synthetic material. The synthetic materials may be polyolefins. Thus, it is able to reduce the immune response of the human body to the implanted part 2, and the service life of the implanted part 2 can be extended.

Additionally, in some examples, the semi-permeable membrane 140 may also have biocompatibility. Thus, the use of the biocompatible membrane 150 can be avoided, and the manufacturing cost can be reduced.

In some examples, a nanoparticle layer 120 is provided on the base layer 110 of the working electrode 10 used for facilitating an analyte enzyme-catalyzed analyte reaction, on basis of which the analyte enzyme sensing layer 130 is formed and then a semi-permeable membrane 140 coating layer is formed on the analyte enzyme sensing layer 130, and finally a biocompatible membrane 150 layer is formed on the semi-permeable membrane 140 coating layer (see Fig. 3). Thus, the service life of the implanted part 2 is extended, interference from other factors is reduced, and the response speed of the implanted part 2 to the analyte is increased.

In some examples, as described above, the implanted part 2 may include a counter electrode 30 (see Fig. 2). In some examples, the counter electrode 30 may be made of platinum, silver, silver chloride, palladium, titanium, or iridium. Thus, the electrochemical reaction at the working electrode 10 cannot be affected while with good electrical conductivity. While the present embodiment is not limited thereto, in other examples, the counter electrode 30 may also be made of at least one selected from gold, glassy carbon, graphite, silver, silver chloride, palladium, titanium, and iridium. Thus, it is able to reduce the influence on the working electrode 10 with good electrical conductivity.

In some examples, the implanted part 2 of the present embodiment may be implemented to be continuously monitoring, thereby continuous monitoring of analyte concentration values in the human body over a long period of time can be achieved (e.g., 1 day to 24 days).

In some examples, as described above, the analyte sensor 1 also includes an application part 3 (see Fig. 1 and Fig. 2).

In some examples, the application part 3 may have a housing 31 (see Fig. 1). In some examples, the temperature sensor 4 provided in the application part 3 may be located inside the housing 31 (see Fig. 1).

In some examples, the temperature sensor 4 may be disposed on an inner wall surface of the housing 31 close to the body surface (see Fig. 1). In other examples, the temperature sensor 4 may be disposed on any wall surface of the housing 31.

In some examples, the number of temperature sensors 4 of the application part 3 may be one. In other examples, the number of the temperature sensors 4 of the application part 3 may be plural, which can improve the accuracy of the body surface temperature sensing, thereby improving the accuracy of the subcutaneous temperature obtained based on the body surface temperature.

In some examples, the application part 3 may be connected to the implanted part 2. In some examples, a part of the implanted part 2 located on the body surface may be electrically connected to the application part 3 via a contact 40 (see Fig. 2). Thus, the current signals generated by the implanted part 2 can be transmitted to the application part 3 by the base layer 110 and the transmission lead via the contact 40.

In some examples, the application part 3 may be made of a flexible PCB and a flexible battery. Thus, it is able to cling to the skin and reduce the influence on the user's daily life.

In some examples, as described above, the analyte sensor 1 further includes a processing module (not shown).

In some examples, the processing module may be mounted on the application part 3. Thus, the current signals generated by the implanted part 2 can be transmitted to the processing module for analysis via the contact 40, and the body surface temperature output by the temperature sensor 4 can be transmitted to the processing module for analysis.

In some examples, the processing module may store a first mapping relationship between the subcutaneous temperature and the body surface temperature. In some examples, as described above, the body surface temperature may be obtained by the temperature sensor 4. In some examples, the processing module may store sensitivity information of the implanted part 2. In some examples, the processing module may store a second mapping relationship between the response signals output by the implanted part 2 and a change in the analyte concentration. In particular, the second mapping relationship may be a second mapping relationship between the response signals output by the implanted part 2 and a change in the analyte concentration at a predetermined temperature.

In some examples, as described above, the processing module may store a first mapping relationship between the subcutaneous temperature and the body surface temperature obtained by the temperature sensor 4.

Fig. 4 is a diagram showing a first mapping relationship between a subcutaneous temperature and a body surface temperature obtained by a temperature sensor 4 according to an embodiment of the present disclosure.

In some examples, three temperature sensors 4 having the same process parameters may be separately placed respectively in the external environment, on the surface of a phantom, and in the depth of 10mm of the phantom subcutaneously. In some examples, with the temperature of the external environment changed, the three temperature sensors 4 may be configured to output corresponding sensed temperatures every 1 minute. The first mapping relationship between the body surface temperature and the subcutaneous temperature can be obtained by respectively outputting the temperature of the external environment, the body surface temperature and the subcutaneous temperature from the three temperature sensors 4 (see Fig. 4).

Wherein the temperature sensor 4 with the same process parameter may refer to temperature sensors 4 delivered from the same batch at the time of production, and may generally be temperature sensors 4 prepared from the same batch of products under the same process. The systematic errors of the measurements between the different temperature sensors 4 can thereby be reduced.

In some examples, as described above, the processing module may store sensitivity information of the implanted part 2.

In some examples, the sensitivity information may be the relationship between the sensitivity of the implanted part 2 and temperature. Specifically, in some examples, the sensitivity of the implanted part 2 may be a change value in the sensitivity of the implanted part 2 at a reference temperature.

In some examples, a change value of the sensitivity of the implanted part 2 may be obtained from the relationship between the response current of the implanted part 2 and the temperature at the reference temperature.

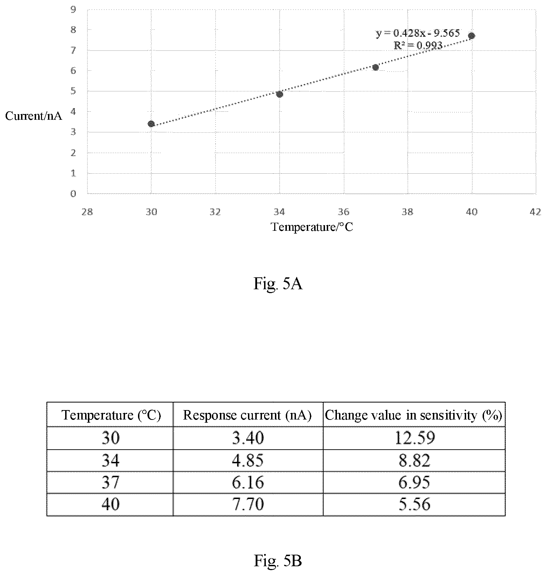

Fig. 5A is a diagram showing results of a linear regression simulation of response current versus temperature for an implanted part 2 according to an embodiment of the present disclosure. Fig. 5B is a table corresponding to the linear regression simulation result diagram in Fig. 5A.

In some examples, the analyte sensor 1 may sense the concentration of glucose. The implanted part 2 of the analyte sensor 1 is placed in a glucose solution. In some examples, the concentration of the glucose solution can be ranged from 5 mmol/Lto 25 mmol/L. In some examples, the temperature of the glucose solution is changed to obtain response signals output from the implanted part 2 when the temperature of the glucose solution is 30°C, 34°C, 37°C, and 40°C (see Fig. 5B). In some examples, the response signals may be response currents (see Fig. 5B). In other examples, the response signals may be response voltages.

In some examples, the relationship of response current and temperature is analyzed, and response current and temperature may be linearly related (see Fig. 5A). In other examples, the relationship between the response current and the temperature is analyzed, and the response current and the temperature may be non-linearly related.

In some examples, the change value of the sensitivity of the implanted part 2 at the reference temperature may be a ratio of a slope (k value) of the linear regression equation of the linear simulation result to a response signal (see Fig. 5A and Fig. 5B in combination). For example, in conjunction with Figs. 5A and 5B, when 30°C is selected as the reference temperature, the change value of the sensitivity of the implanted part 2 at 30°C may be 12.59% (0.428/3.40). When 34°C is selected as the reference temperature, the change value of the sensitivity of the implanted part 2 at 34°C is 8.82% (0.428/4.85). When 37°C is selected as the reference temperature, the change value of the sensitivity of the implanted part 2 at 37°C is 6.95% (0.428/6.16). When 40°C is selected as the reference temperature, the change value of the sensitivity of the implanted part 2 at 40°C is 5.56% (0.428/7.70).

In some examples, the change value in sensitivity of the implanted part 2 at the reference temperature may be an average of the results of multiple repeated measurements of the same implanted part 2. In other examples, the change value in sensitivity of the implanted part 2 at the reference temperature may be an average of measurement results of a plurality of implanted part 2. In both above two cases, through the calculation of the average, the systematic errors can be reduced and the accuracy of the calculation result can be improved. Thus, it is facilitated to improve the accuracy of the obtained analyte concentration.

In some examples, as described above, the processing module may store a second mapping relationship of variation of the response signals of the implanted part 2 over analyte concentration at a predetermined temperature.

In some examples, the predetermined temperature includes a plurality of temperature values. For example, in some examples, the predetermined temperature includes 34°C, 35°C, 36°C, 36.5°C, 37°C, 37.5°C, 38°C, 39°C, 40°C, and 41°C. In some examples, the reference temperature may be selected from one of a plurality of temperature values. For example, in some examples, the reference temperature can be 34°C, 35°C, 36°C, 36.5°C, 37°C, 37.5°C, 38°C, 39°C, 40°C, or 41°C.

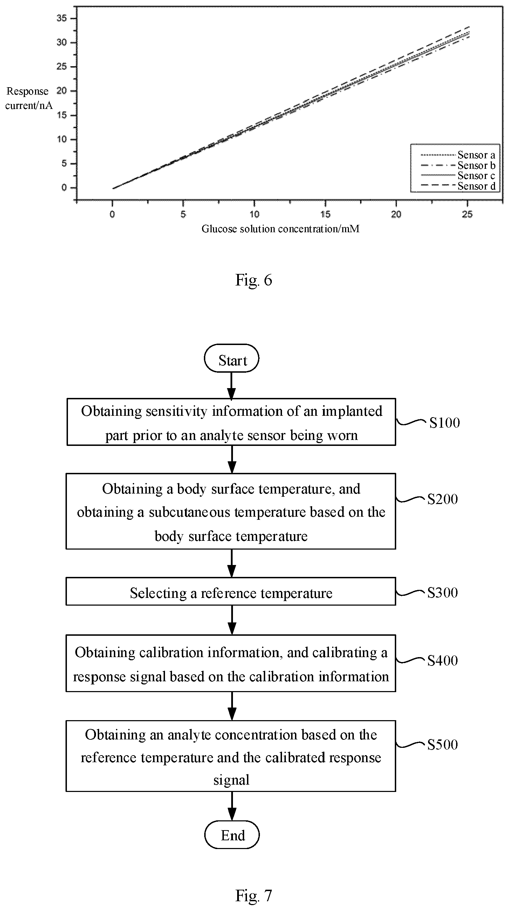

Fig. 6 is a diagram showing a second mapping relationship between response current and analyte concentration according to an embodiment of the present disclosure.

In some examples, the analyte sensor 1 may sense the concentration of glucose. 37°C is selected as the reference temperature. The implanted parts 2 of the analyte sensor 1 are placed in glucose solutions of different concentrations respectively. In some examples, the concentration of the glucose solution can be 0 mmol to 25 mmol (see Fig. 6). The implanted part 2 senses glucose solutions of different concentrations and outputs corresponding response signals. In some examples, the response signals may be response currents (see Fig. 6). In other examples, the response signals may be response voltages.

In some examples, the relationship between the response current and the analyte concentration is analyzed. The response current and glucose concentration may be linearly related (see Fig. 6), i.e., the second mapping relationship is a linear relationship. In other examples, the relationship between the response current and the analyte concentration is analyzed. The response current and the analyte concentration may be non-linearly related, i.e., the second mapping relationship is a non-linear relationship.

In some examples, the processing module is configured for obtaining a subcutaneous temperature. For example, the processing module may be configured for obtaining the subcutaneous temperature based on the body surface temperature and the first mapping relationship. In some examples, the processing module is configured for selecting a reference temperature. In some examples, the processing module is configured for obtaining calibration information. For example, the processing module is configured for obtaining the calibration information based on the sensitivity information, the subcutaneous temperature, and the reference temperature. In some examples, the processing module is configured for calibrating a response signal. For example, the processing module is configured for calibrating the response signal obtained by the implanted part 2 based on the calibration information. In some examples, the processing module is configured for obtaining an analyte concentration. For example, the processing module is configured for obtaining the analyte concentration based on the reference temperature, the calibrated response signal, and the second mapping relationship.

In some examples, as described above, the processing module may be configured for obtaining the subcutaneous temperature based on the body surface temperature and the first mapping relationship. Specifically, the temperature sensor 4 can transmit the sensed body surface temperature to the processing module. The processing module obtains the subcutaneous temperature based on the body surface temperature and the first mapping relationship according to the first mapping relationship preset in the processing module.

In some examples, the processing module is configured for selecting a reference temperature, as described above. In some examples, the reference temperature selected by the process module may be 37°C. In this case, the reference temperature is relatively close to the average body temperature of the human body, whereby the effect of calibrating the analyte concentration with the temperature compensation considered can be improved. In other examples, the reference temperature may also be other temperature. For example, the reference temperature can be 35°C, 36°C, 36.5°C, 37.5°C, or 38°C, etc.

In some examples, as described above, the processing module is configured to obtain calibration information based on the sensitivity information, the subcutaneous temperature, and the reference temperature. Specifically, in some examples, the processing module is configured for calculating a difference between the reference temperature and the subcutaneous temperature and then calculating a product of the difference and the change value in sensitivity of the implanted part 2 at 37°C to obtain calibration information.

In some examples, as described above, the processing module is configured for calibrating the response signals obtained by the implanted part 2 based on the calibration information. Specifically, in some examples, the response signals generated by the implanted part 2 can be delivered by the contact 40 to a processing module configured for performing mathematical calculations on the calibration information and the response signals to calibrate the response signals obtained by the implanted part 2.

In some examples, the calibration formula for the response signals obtained by the implanted part 2 may be b=a(1+ΔT×Z). Wherein ΔT represents a difference between the reference temperature and the subcutaneous temperature, Z represents a change value of the sensitivity of the implanted part 2 at the reference temperature, a represents the response signal output by the implanted part 2 to the processing module, and b represents the calibrated response signal.

In some examples, as described above, the processing module is configured for obtaining the analyte concentration based on the reference temperature, the calibrated response signal, and the second mapping relationship. Specifically, in some examples, the processing module is configured for calculating a calibrated analyte concentration with a second mapping relationship at a reference temperature preset by the processing module and a calibrated response signal.

In some examples, the temperature sensor 4 and the implanted part 2 (particularly on empty stomach and for a period of time after a meal) may transmit signals to the processing module at intervals. The processing module may output calibrated analyte concentrations to the outside at intervals, so that a user may know a trend of change in the analyte concentration in time, thereby controlling the change in the analyte concentration.

In some examples, the analyte concentration signals obtained by the processing module may be transmitted via wireless communication, for example, Bluetooth, WIFI, etc. An external reader device, for example a cell phone, a computer (not shown) may receive the analyte concentration signals sent by the processing module and display the analyte concentrations.

In the analyte sensor 1 according to the present disclosure, the implanted part 2 is placed subcutaneously and the application part 3 having the temperature sensor 4 is placed on the body surface, body surface temperature is obtained by the temperature sensor 4, and subcutaneous temperature is obtained based on the body surface temperature. The processing module is configured as followed: obtaining a subcutaneous temperature based on the body surface temperature and the first mapping relationship, selecting a reference temperature, obtaining calibration information based on the sensitivity information, the subcutaneous temperature, and the reference temperature, calibrating a response signal obtained from the implanted part based on the calibration information, and obtaining the analyte concentration based on the reference temperature, the calibrated response signal, and the second mapping relationship. It is thereby able to obtain the analyte concentration in consideration of the temperature compensation, improving the accuracy with which the analyte sensor 1 senses the analyte concentration.

Hereinafter, in conjunction with the analyte sensor 1 described above, a method for obtaining an analyte concentration with temperature compensation considered according to the present disclosure is described.

The method for obtaining the analyte concentration with temperature compensation considered according to the present embodiment may also be referred to as a calibration method for the analyte concentration, a method for the analyte concentration calibration with the temperature compensation, a temperature compensation calibration method for the analyte concentration, etc. It should be noted that each of the names is intended to indicate a method according to the present embodiment capable of improving the accuracy of the obtained analyte concentration with temperature compensation considered, and should not be understood to be limiting.

Fig. 7 is a flowchart showing a method for obtaining an analyte concentration with temperature compensation considered according to an embodiment of the present disclosure.

Referring to Fig. 7, the method for obtaining an analyte concentration with temperature compensation considered according to the present disclosure may include: obtaining sensitivity information of the implanted part 2 prior to the analyte sensor 1 being worn (step S100); obtaining a body surface temperature, and obtaining a subcutaneous temperature based on the body surface temperature (step S200); selecting a reference temperature (step S300); obtaining calibration information, and calibrating a response signal based on the calibration information (step S400); and obtaining the analyte concentration based on the reference temperature and the calibrated response signal (step S500).

In step S 100, the sensitivity information of the implanted part 2 may be obtained before the analyte sensor 1 is worn, as described above. In some examples, the sensitivity information of the implanted part 2 may be obtained at a predetermined analyte concentration prior to the analyte sensor 1 being worn.

In some examples, in step S 100, the predetermined analyte concentration may be a known and the same analyte concentration. In some examples, the implanted part 2 may be placed in an analyte and a sensed response signal of the analyte concentration is output by the implanted part 2 to obtain sensitivity information of the implanted part 2.

In some examples, in step S 100, the sensitivity information may be the relationship between the sensitivity of the implanted part 2 and temperature.

In some examples, in step S 100, the sensitivity of the implanted part 2 may increase as the temperature increases. Specifically, in some examples, the sensitivity of the implanted part 2 may increase as the temperature increases within a predetermined temperature range. Wherein, in some examples, the sensitivity of the implanted part 2 may be linearly related to temperature. In other examples, the sensitivity of the implanted part 2 may also be non-linearly related to the temperature.

In other examples, in step S100, the sensitivity of the implanted part 2 may decrease as the temperature increases. Specifically, in some examples, in step S100, the sensitivity of the implanted part 2 may decrease as the temperature increases within a predetermined temperature range. Wherein, in some examples, the sensitivity of the implanted part 2 may be linearly related to temperature. In other examples, the sensitivity of the implanted part 2 may also be non-linearly related to the temperature.

In some examples, as previously described, the sensitivity information may be a change value of the sensitivity of the implanted part 2 at a reference temperature. For example, in some examples, as previously described, the sensitivity information may be a change value in the sensitivity of the implanted part 2 at 37°C, i.e., 6.95%.

In some examples, in step S 100, the implanted part 2 may be placed in a reagent containing an analyte, the sensitivity information of the implanted part 2 may be obtained with the temperature of the reagent changed and the sensitivity of the implanted part 2 over the temperature of the reagent measured. That is, the temperature of the environment (position) located by the implanted part 2 is changed through the change of temperature of the reagent to obtain the relationship between the sensitivity of the implanted part 2 and the temperature of the environment (location). In this case, prior to the analyte sensor 1 being worn, the reagent containing an analyte is used to measure the variation relationship of sensitivity of the implanted part 2 over temperature, thus it is facilitated for the sensitivity information of the implanted part 2 to be obtained in advance

In some examples, in step S100, the concentration of the analyte in the reagent is kept constant when the temperature of the reagent is changed. In this case, when the temperature of the reagent is changed, the change in the temperature does not affect the change in the analyte concentration. Thus, the accuracy of sensitivity inspection of the implanted part 2 can be improved.

In step S200, a body surface temperature is obtained as described above, and a subcutaneous temperature is obtained based on the body surface temperature. Specifically, in some examples, in step S200, the implanted part 2 may be placed subcutaneously and the application part 3 may be placed on the body surface. The body surface temperature may be obtained by the temperature sensor 4, and the subcutaneous temperature may be obtained based on the body surface temperature.

In some examples, in step S200, when the application part 3 is placed on the body surface, the temperature sensor 4 that the application part 3 has is placed on the body surface. Thus, the temperature sensor 4 can sense the temperature of the body surface and output the body surface temperature.

In some examples, in step S200, as previously described, there may be a first mapping relationship between the body surface temperature and the subcutaneous temperature. When the body surface temperature is obtained, the subcutaneous temperature can be obtained through the first mapping relationship.

In some examples, in step S200, the body surface temperature and the subcutaneous temperature of the phantom may be simultaneously sensed at different environment temperatures to thereby obtain the first mapping relationship between the body surface temperature and the subcutaneous temperature.

In some examples, in step S200, the body surface temperature and the subcutaneous temperature are jointly influenced by the environment temperature and the body temperature. The body surface temperature is influenced by the environment temperature to the extent more than the subcutaneous temperature. The body surface temperature is influenced by the body temperature to the extent less than the subcutaneous temperature. Thus, there may be a non-linear correlation between body surface temperature and subcutaneous temperature. That is, the first mapping relationship may be a non-linear mapping relationship.

In some examples, in step S200, the implanted part 2 may be placed at a depth of 3 mm to 20 mm subcutaneously. It should be understood that the subcutaneous distance between 3 mm and 20 mm is small and the temperatures are approximately equal, which will not result in a statistical difference in the response signal output by the implanted part 2.

In step S300, the reference temperature may be selected as described above.

In some examples, 37°C may be selected as a reference temperature in step S300. In this case, the reference temperature is relatively close to the average body temperature of the human body, so that the effect of calibrating the analyte concentration by temperature compensation can be improved. In other examples, the reference temperatures may also be other temperatures. For example, the reference temperature can be 35°C, 36°C, 36.5°C, 37.5°C, or 38°C, etc.

In step S400, as described above, the calibration information may be obtained based on the sensitivity information, the reference temperature and the subcutaneous temperature. The response signal obtained by the implanted part 2 may be calibrated based on the calibration information.

In some examples, in step S400, the sensitivity information may be the relationship between the sensitivity of the implanted part 2 and the temperature change as described above. In some examples, particularly, the sensitivity information may be the sensitivity of the implanted part 2 at a reference temperature. Further, the sensitivity information may be a change value in the sensitivity of the implanted part 2 at a reference temperature.

In some examples, in step S400, the calibration information may be obtained based on the difference between the subcutaneous temperature and the reference temperature, and the sensitivity information. In this case, the temperature compensation can be facilitated by considering the relationship between the sensitivity of the implanted part 2 and the temperature, and the relationship between the subcutaneous temperature at which the implanted part 2 is located and the reference temperature.

Specifically, in some examples, the calibration information may be product of a difference between the reference temperature and the subcutaneous temperature multiplied by the sensitivity of the implanted part 2 at the reference temperature. Further, the calibration information may be product of a difference between the reference temperature and the subcutaneous temperature multiplied by a change value in sensitivity of the implanted part 2 at the reference temperature.

In other examples, in step S400, the calibration information may be obtained based on sensitivity information, and a ratio of a subcutaneous temperature to a reference temperature. Specifically, in some examples, the calibration information may be product of a ratio of the reference temperature to the subcutaneous temperature multiplied by the sensitivity of the implanted part 2 at the reference temperature. Further, the calibration information may be a product of a ratio of the reference temperature to the subcutaneous temperature multiplied by a change value in sensitivity of the implanted part 2 at the reference temperature.

In some examples, in step S400, the calibrated response signal may be a sum of a product of a pre-calibration response signal multiplied by the calibration information, then plus the pre-calibration response signal. That is, the calibrated response signal may be a product of a sum of calibration information plus one multiplied by the pre-calibration response signal.

In other examples, in step S400, the calibrated response signal may be a quotient of the pre-calibration response signal divided by the calibration information. In still other examples, in step S400, the calibrated response signal may be a sum/difference of the pre-calibration response signal and the calibration information.

In some examples, in step S400, when the temperature sensor 4 senses and outputs the temperature of the body surface, the implanted part 2 inserted subcutaneously may simultaneously sense the analyte concentration subcutaneously to output a response signal. In this case, the subcutaneous temperature, i.e., the temperature at the location of the implanted part 2, and the response signal output by the implanted part 2 sensing the analyte concentration can be simultaneously obtained, thus, the temperature compensation can be accomplished for the response signal of the implanted part 2 in real time to improve the accuracy of the analyte concentration calibration.

In some examples, in step S400, there is no time delay between the output of the response signal from the implanted part 2 and the output of the body surface temperature from the temperature sensor 4. In this case, the subcutaneous temperature (that is, the temperature at the position where the implanted part 2 is located) is obtained based on the body surface temperature, and the response signal output by the implanted part 2 at this subcutaneous temperature can be obtained without a time delay, so that the accuracy of the analyte concentration calibration can be further improved.

In step S500, as described above, the analyte concentration may be obtained based on the reference temperature and the calibrated response signal.

In some examples, in step S500, the relationship between the response signal of the implanted part 2 and the analyte concentration at the reference temperature can be obtained. In some examples, there may be a second mapping relationship between the response signal of the implanted part 2 and the concentration of the analyte at the reference temperature.

In some examples, in step S500, the response signal of the implanted part 2 may be linearly related to the analyte concentration. That is, the second mapping relationship may be a linear mapping relationship. In other examples, the response signal of the implanted part 2 may be non-linearly related to the analyte concentration. That is, the second mapping relationship may be a non-linear mapping relationship.

In some examples, in step S500, the implanted parts 2 are placed in analyte solutions of different concentrations at a reference temperature respectively. The response signals output by the implanted parts 2 are measured to obtain a second mapping relationship between the response signal of the implanted parts 2 and the analyte concentration.

In some examples, in step S500, a maximum concentration of the analyte for inspecting the second mapping relationship is lower than a maximum sensed concentration of the implanted part 2. In some examples, the concentration gradient of the analyte used for obtaining the second mapping relationship increases progressively. In some examples, the concentration of the analyte used for inspection to obtain the second mapping relationship approximates the concentration of the subcutaneous analyte. In this case, the proximity of the concentration of the analyte may enable the systematic error of the inspection to be relatively small, thereby resulting in improvement of the accuracy of the inspection.

In some examples, in step S500, a calibrated analyte concentration may be obtained based on the relationship between the response signal of the implanted part 2 and the analyte concentration change at the reference temperature and the calibrated response signal. That is, it is able to obtain a calibrated analyte concentration through the second mapping relationship and the response signal of the implanted part 2 calibrated in step S400.

In the method according to the present disclosure, prior to the analyte sensor 1 being worn, the sensitivity information of the implanted part 2 is obtained at a predetermined analyte concentration. That is, the relationship between the sensitivity of the implanted part 2 and the temperature change is obtained. An implanted part 2 is placed subcutaneously and an application part 3 having a temperature sensor 4 is placed on the body surface. A body surface temperature is obtained by the temperature sensor 4, and a subcutaneous temperature is obtained based on the body surface temperature. The reference temperature is selected, the calibration information is obtained based on the sensitivity information, the reference temperature and the subcutaneous temperature, and the response signal obtained by the implanted part 2 is calibrated based on the calibration information, whereby the calibrated response signal can be obtained. Based on the reference temperature and the calibrated response signal, the analyte concentration is calibrated, whereby the accuracy of the obtained analyte concentration can be improved.

While the present disclosure has been particularly shown and described with reference to the accompanying drawings and examples, it should be understood that the disclosure is not limited in any manner by the foregoing description. Modifications and variations of the present disclosure, as required, may be made by those skilled in the art without departing from the true spirit and scope and fall within the scope of the disclosure.

## Claims

1. A method for obtaining analyte concentration with temperature compensation considered, the analyte concentration being obtained by an analyte sensor, the analyte sensor comprising an implanted part that is capable of being placed subcutaneously and an application part that is capable of being placed on a body surface and has a temperature sensor, **characterized in that** the method comprises: obtaining sensitivity information of the implanted part at a predetermined analyte concentration prior to the analyte sensor being worn, the sensitivity information is a relationship between the temperature and the sensitivity of the implanted part; placing the implanted part subcutaneously and the application part on a body surface, obtaining a body surface temperature via the temperature sensor, and obtaining a subcutaneous temperature based on the body surface temperature; selecting a reference temperature; obtaining calibration information based on the sensitivity information, the reference temperature, and the subcutaneous temperature, and calibrating a response signal obtained by the implanted part based on the calibration information; and obtaining the analyte concentration based on the reference temperature and the calibrated response signal.

2. The method of claim 1, **characterized in that**
placing the implanted part in a reagent containing the analyte, changing a temperature of the reagent, and measuring a variation of sensitivity of the implanted part over the temperature of the reagent to obtain sensitivity information of the implanted part.

3. The method of claim 2, **characterized in that**
Keeping the concentration of the analyte in the reagent to be constant when the temperature of the reagent is changed.

4. The method of claim 1, **characterized in that**
Obtaining the calibration information based on the sensitivity information and a difference or ratio of the subcutaneous temperature to the reference temperature.

5. The method of claim 1, **characterized in that**
When the temperature sensor senses a temperature on the body surface and outputs the body surface temperature, the implanted part inserted subcutaneously senses a subcutaneous analyte concentration simultaneously and outputs a response signal.

6. The method of claim 5, **characterized in that**
There is no time delay between the output of the response signal by the implanted part and the output of the body surface temperature by the temperature sensor.

7. The method of claim 1, **characterized in that**
The analyte is one or more of acetylcholine, amylase, bilirubin, cholesterol, chorionic gonadotropin, creatine kinase, creatine, creatinine, DNA, fructosamine, glucose, glutamine, growth hormone, hormone, ketone body, lactate, peroxide, prostate specific antigen, prothrombin, RNA, thyroid stimulating hormone, and troponin.

8. The method of claim 1, **characterized in that**
Obtaining a calibrated analyte concentration based on the relationship between the response signal of the implanted part and the change in analyte concentration at the reference temperature and a calibrated response signal.

9. An analyte sensor for obtaining an analyte concentration with temperature compensation considered, **characterized in that** the analyte sensor comprises an implanted part capable of being placed subcutaneously, an application part capable of being placed on a body surface and having a temperature sensor, and a processing module storing a first mapping relationship between the subcutaneous temperature and the temperature of the body surface obtained by the temperature sensor, sensitivity information of the implanted part, and a second mapping relationship between a response signal output by the implanted part and a change in the analyte concentration at a predetermined temperature, wherein the sensitivity information is a relationship between a sensitivity of the implanted part and a temperature change at a predetermined analyte concentration; when the implanted part is placed subcutaneously and the application part is placed on the body surface, the temperature sensor senses the temperature of the body surface and outputs the temperature of the body surface; and the processing module is configured for obtaining a subcutaneous temperature based on the body surface temperature and the first mapping relationship, selecting a reference temperature, obtaining calibration information based on the sensitivity information, the subcutaneous temperature, and the reference temperature, calibrating a response signal obtained by the implanted part based on the calibration information, and obtaining the analyte concentration based on the reference temperature, the calibrated response signal, and the second mapping relationship.

10. The analyte sensor of claim 9, **characterized in that**
the implanted part includes a working electrode capable of reacting with an analyte, and a counter electrode forming a loop with the working electrode.
